# EUROPEAN PATENT APPLICATION

(11) **EP 2 072 507 A1**
(43) Date of publication of application: **24.06.2009**
(21) Application number: 07829151.5
(22) Date of filing: 27.09.2007
(51) Int. Cl.: C07D 313/12, C07B 61/00

(54) **TERTIARY ALKYL ESTER OF OXODIBENZOXEPIN ACETIC ACID**

(30) Priority: 02.10.2006 JP 2006271311
(71) Applicant: Sumitomo Chemical Company, Limited, Tokyo 104-8260 (JP)
(72) Inventor: KATSURA, Tadashi, Toyonaka-shi, Osaka 560-0013 (JP); HAYASHI, Taketo, Sanda-shi, Hyogo 669-1547 (JP); TANAKA, Masahide, Nishinomiya-shi, Hyogo 663-8105 (JP)
(74) Representative: Kuhnen & Wacker
(86) International application number: PCT/JP2007/069412
(87) International publication number: WO 2008/041734

(57) **Abstract**

A tertiary alkyl ester represented by Formula (2): 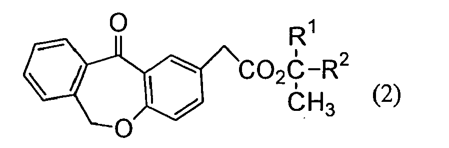 wherein R¹ and R² each independently represent a C₁₋₄ alkyl group, and a method for producing the same.

## Description

### Technical Field

The present invention relates to a tertiary alkyl ester of oxodibenzoxepinacetic acid useful as an intermediate for preparing pharmaceuticals, and a method for producing the same.

### Background Art

Dibenzoxepinacetic acid derivatives are useful compounds in pharmaceutical field. For example, with respect to olopatadine, a medicament useful as an antiallergic drug, a production method is known which includes steps of performing a reaction of a dibenzoxepinacetic acid derivative with 2-amino-2-methyl-1-propanol to produce an oxazoline derivative, and then performing a reaction with 3-dimethylaminopropylmagnesium chloride, according to the following scheme (See JP-B-7-116174).

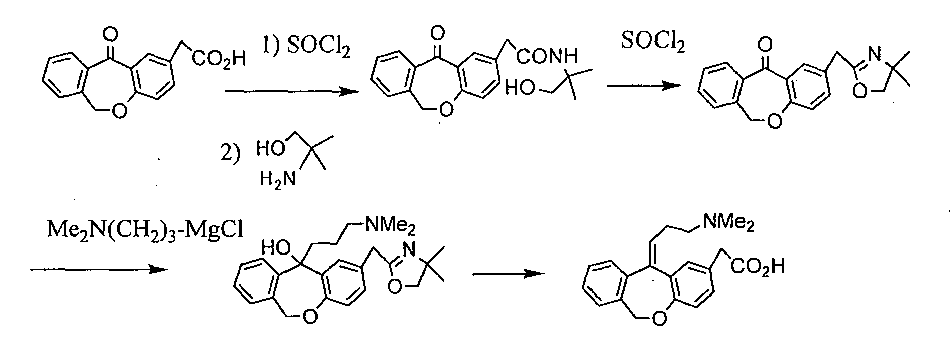

A method for producing olopatadine according to the scheme below is also known, which includes steps of reducing a dibenzoxepinacetic acid derivative to produce a dibenzoxepinethanol derivative, protecting the hydroxy group, and then performing a reaction with 3-dimethylaminopropylmagnesium chloride (See JP-B-5-86925).

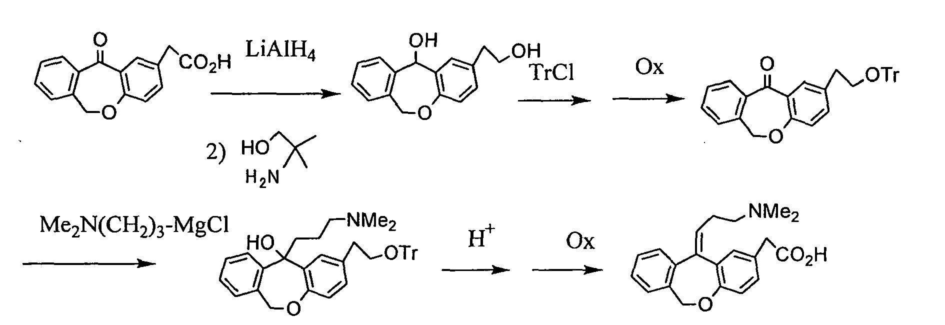

Both of the above production methods are problematic in that bothersome processes are required for the production of the dibenzoxepin derivatives that are to be reacted with 3-dimethylaminopropylmagnesium chloride.

### Disclosure of the Invention

Accordingly, if a dibenzoxepin derivative that can be easily produced and can be converted to olopatadine by a reaction with 3-dimethylaminopropylmagnesium chloride was found, olopatadine could be manufactured economically.

The invention is based on the finding that a tertiary alkyl ester of oxodibenzoxepinacetic acid solves the above problems.

Specifically, the invention relates to:
[1] A tertiary alkyl ester represented by Formula (2): 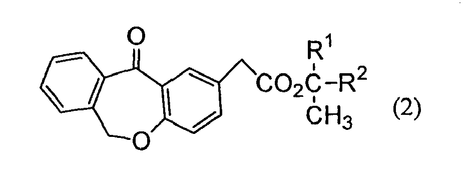 wherein R¹ and R² each independently represent a C₁₋₄ alkyl group,
[2] A tertiary alkyl ester according to [1], wherein R¹ and R² each represent a methyl group (a C₁ alkyl group),
[3] A method for producing a tertiary alkyl ester represented by Formula (2): 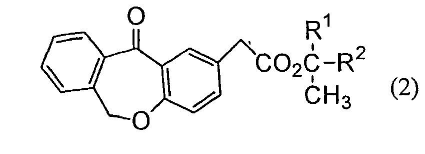 wherein R¹ and R² each independently represent a C₁₋₄ alkyl group, the method comprising esterifying a carboxylic acid represented by Formula (1): 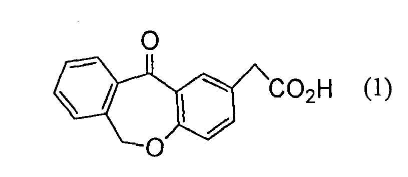 with trifluoroacetic anhydride and a tertiary alcohol,
[4] A method for producing a tertiary alkyl ester represented by Formula (2): 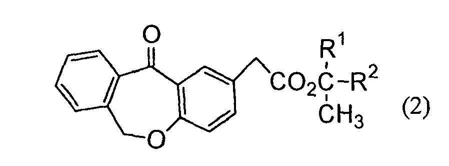 wherein R¹ and R² each independently represent a C₁₋₄ alkyl group, the method comprising esterifying a carboxylic acid represented by Formula (1): 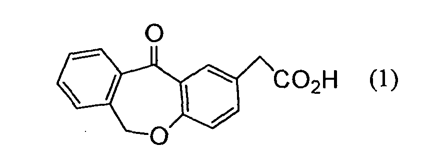 with a vinylidene compound represented by Formula (4): 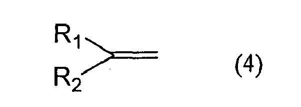 wherein R¹ and R² each independently represent a C₁₋₄ alkyl group, and a phosphorus halide,
[5] A method for producing a tertiary alkyl ester represented by Formula (2): 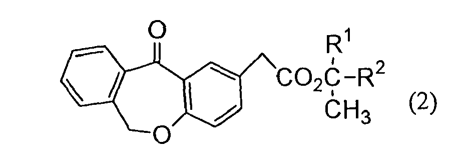 wherein R¹ and R² each independently represent a C₁₋₄ alkyl group, the method comprising:
   [Step 1] a step of a reaction of phthalide with thionyl chloride to produce 2-chloromethylbenzoyl chloride;
   [Step 2] a step of a reaction of the 2-chloromethylbenzoyl chloride with methanol to produce methyl 2-chloromethylbenzoate represented by Formula (5): 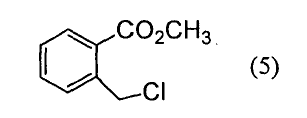
   [Step 3] a step of a reaction of the methyl 2-chloromethylbenzoate with methyl 4-hydroxyphenylacetate represented by Formula (6): 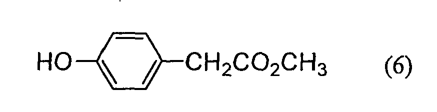 to produce methyl 2-(4-methoxycarbonylmethylphenoxymethyl)benzoate represented by Formula (7): 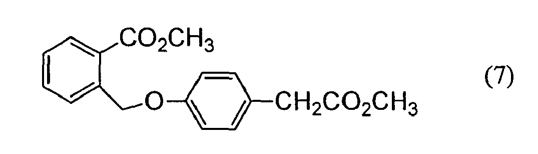
   [Step 4] a step of hydrolyzing the methyl 2-(4-methoxycarbonylmethylphenoxymethyl)benzoate to produce 2-(4-carboxymethylphenoxymethyl)benzoic acid represented by Formula (8): 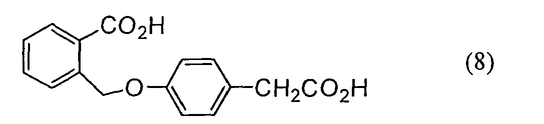
   [Step 5] a step of treating the 2-(4-carboxymethylphenoxymethyl)benzoic acid with trifluoroacetic anhydride or a halogenating agent and cyclizing with a Lewis acid catalyst to produce a carboxylic acid represented by Formula (1); and
   [Step 6] esterifying the carboxylic acid represented by Formula (1) by a method described in [3] or [4], and
[6] A method for producing olopatadine, the method comprising steps of a reaction of a t-butyl ester represented by Formula (3): 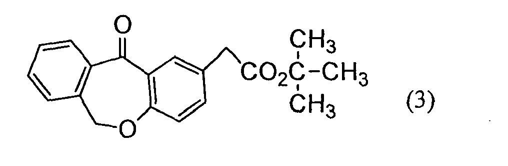 with 3-dimethylaminopropylmagnesium chloride to produce t-butyl 11-hydroxy-11-(3'-dimethylaminopropyl)-6,11-dihydrodibenz[b,e]oxepin-2-acetate represented by Formula (9): 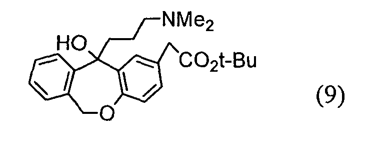 and then subjecting the same to dehydration reaction and de-esterification reaction.

A tertiary alkyl ester represented by Formula (2): 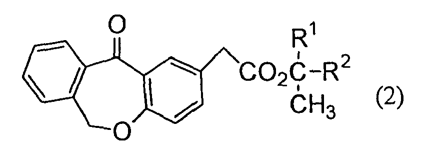 of the present invention, wherein R¹ and R² each independently represent a C₁₋₄ alkyl group, can be produced by esterifying a carboxylic acid represented by Formula (1) :

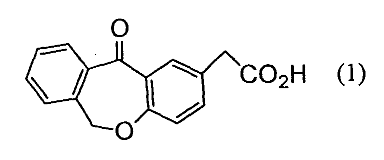

Specific examples of the alkyl groups represented by R¹ and R² include a methyl group, an ethyl group, a propyl group, an isopropyl group and n-butyl group.

Both of R¹ and R² are methyl groups in a preferable example of the tertiary alkyl ester represented by Formula (2), and the example is a t-butyl ester represented by the following Formula (3): 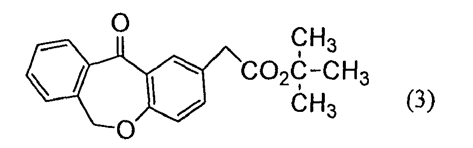 that is t-butyl 11-oxo-6,11-dihydrodibenz[b,e]oxepin-2-acetate.

Esterification may be performed with, for example, trifluoroacetic anhydride and a tertiary alcohol represented by Formula (10): 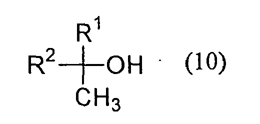 wherein R¹ and R² each independently represent a C₁₋₄ alkyl group. More specifically, esterification reaction can be performed as follows. First, trifluoroacetic anhydride is added to a carboxylic acid represented by Formula (1) in an amount of about 1 to 1.5 mol per 1 mol of the carboxylic acid, and stirred at a temperature of about 0 to 50°C for about 0.5 to 2 hours. Subsequently, a sufficient amount of a tertiary alcohol represented by Formula (10) is added thereto, and stirred at temperature of about 0 to 100°C for about 0.5 to 10 hours.

Specific examples of the tertiary alcohols represented by Formula (10) include t-butanol, 2-methylbutan-2-ol and 3-methylpentane-3-ol. Among these, t-butanol is especially preferable. Use of t-butanol provides t-butyl ester represented by the above Formula (3).

As another esterification method, a method using a vinylidene compound represented by Formula (4): 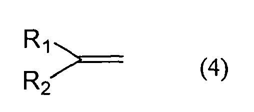 wherein R¹ and R² each independently represent a C₁₋₄ alkyl group, and a phosphorus halide can be mentioned.

In this method, the carboxylic acid represented by Formula (1) and the above vinylidene compound react in the presence of a phosphorus halide.

The amount of the vinylidene compound represented by Formula (4) is not limited, and is preferably about 1.5 to 10 mol, more preferably 1.8 to 5 mol, per 1 mol of the dibenzoxepinacetic acid represented by Formula (1).

Specific examples of the vinylidene compounds represented by Formula (4) include isobutene, 2-methylpropene, 2-methyl-1-butene and 2-methyl-l-pentene. Among these, isobutene is preferable. Use of isobutene provides the t-butyl ester represented by the above Formula (3).

The amount of the phosphorus halide is not limited, and is preferably 0.05 to 1 mol, more preferably 0.15 to 0.4 mol, per 1 mol of carboxylic acid represented by Formula (1).

Examples of the phosphorus halides include phosphorus oxytrichloride, phosphorus trichloride, phosphorus pentachloride and dichlorophosphonic acid. Among these, phosphorus oxytrichloride, phosphorus trichloride, and dichlorophosphonic acid are preferable, and phosphorus oxytrichloride is especially preferable.

More specifically, the procedure may be as follows. A vinylidene compound represented by Formula (4) is dissolved in an inert solvent such as toluene and chlorobenzene, and a carboxylic acid represented by Formula (1) is added thereto. A phosphorus halide is further added thereto, and stirred at a temperature of about 15 to 60°C for about 1 to 24 hours. During this procedure, in terms of promotion of the reaction, a catalytic amount of water is preferably used. The amount of the water is preferably 0.05 to 0.25 mol, more preferably 0.07 to 0.2 mol, per 1 mol of the carboxylic acid represented by Formula (1).

It is also possible to add a carboxylic acid represented by Formula (1), a phosphorus halide, and a catalytic amount of water to an inert solvent such as toluene and chlorobenzene, and successively add a vinylidene compound represented by Formula (4) thereto at a temperature of about 15 to 60°C.

In either of the above esterification methods, after the esterification reaction, the reaction mixture may be suitably subjected to phase separation, washing, drying, isolation, concentration, etc., according to a known method as required, thereby obtaining the target substance.

A carboxylic acid represented by Formula (1) used to produce the tertiary alkyl ester represented by Formula (2) of the invention can be prepared, for example, through the following [Step 1] to [Step 5]. This preparation method gives the carboxylic acid represented by Formula (1) at high yield, and the reactions can be performed at temperatures of less than 140°C. Accordingly, steam heating can be easily employed, which provides convenience.

### [Step 1] Step of a reaction of phthalide with thionyl chloride to produce 2-chloromethylbenzoyl chloride:

In this step, phthalide is dissolved in an inert solvent such as xylene, and thionyl chloride is added thereto while heating in the presence of a catalyst such as BF₃-etherate complex. The amount of the solvent is preferably 100 to 1000 parts by weight per 100 parts by weight of the phthalide. The amount of the catalyst is preferably about 0.01 to 0.2 mol per 1 mol of the phthalide.

The reaction mixture preferably contains a quarternary ammonium salt such as benzyltriethylammonium chloride for the purpose of promoting the reaction. The content thereof is preferably about 0.01 to 0.2 mol per 1 mol of the phthalide.

The amount of the thionyl chloride is preferably about 1 to 2 mol per 1 mol of the phthalide. The solution temperature upon addition of the thionyl chloride to a phthalide solution is preferably 80 to 130°C. Dropwise addition of thionyl chloride is preferably performed over about 0.5 to 2 hours per molar quantity. After the dropwise addition of the thionyl chloride, in order to further ensure the reaction, the mixture is preferably stirred at temperature of about 120 to 135°C for about 1 to 5 hours.

After the reaction is completed, excessive thionyl chloride and solvent are removed. Preferably, the thionyl chloride is distilled off at normal pressure, and the solvent is then distilled off under reduced pressure. As a result, 2-chloromethylbenzoyl chloride is obtained as a residue.

### [Step 2] Step of a reaction of 2-chloromethylbenzoyl chloride with methanol to produce methyl 2-chloromethylbenzoate represented by Formula (5):

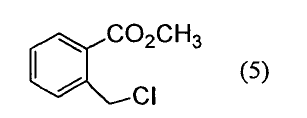

This step may be as follows, for example. Methanol is added dropwise to 2-chloromethylbenzoyl chloride taking about 0.5 to 2 hours per 1 mol of methanol, and stirred at 30 to 60°C for about 0.5 to 1 hour. It is also possible to add 2-chloromethylbenzoyl chloride dropwise to a mixed solution of methanol and toluene. The amount of the methanol is preferably 1 to 3 mol per 1 mol of the 2-chloromethylbenzoyl chloride.

The reaction mixture obtained by the reaction described above contains hydrochloric acid produced during the reaction, and thus is preferably neutralized with alkali. For example, the above reaction mixture may be added dropwise at 0 to 50°C to an aqueous solution having dissolved therein a designated amount of potassium carbonate. After neutralization, washing is performed with brine having a concentration of about 15 to 35 wt%, and the organic layer is concentrated under reduced pressure, thereby giving methyl 2-chloromethylbenzoate represented by Formula (5) as a residue.

### [Step 3] Step of a reaction of methyl 2-chloromethylbenzoate with methyl 4-hydroxyphenylacetate represented by Formula (6):

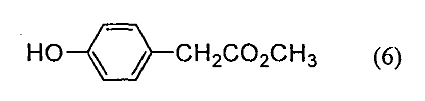

to produce methyl 2-(4-methoxycarbonylmethylphenoxymethyl) benzoate represented by Formula (7):

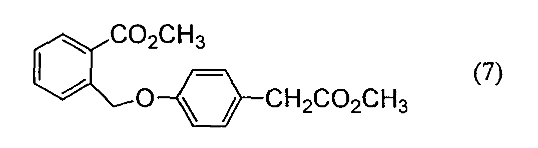

This step may be as follows, for example. A mixture of methyl 2-chloromethylbenzoate, potassium carbonate and a solvent such as dimethylacetamide is heated to a temperature of about 50 to 120°C, a designated amount of methyl 4-hydroxyphenylacetate is added thereto, and the mixture is stirred at the same temperature for about 1 to 12 hours. At that time, the amounts of the methyl 2-chloromethylbenzoate and the methyl 4-hydroxyphenylacetate are preferably approximately stoichiometric amounts (equimolar amounts), and the methyl 4-hydroxyphenylacetate is preferably added dropwise over about 0.5 to 2 hours per 0.2 molar quantity. The amount of the potassium carbonate is preferably about 0.5 to 1.5 mol per 1 mol of the methyl 2-chloromethylbenzoate. The preferable amount of the solvent such as dimethylacetamide is about 100 to 500 parts by weight per 100 parts by weight of the methyl 4-hydroxyphenylacetate.

After the reaction, the target substance may be isolated by a known method. For example, the reaction mixture is poured into water, an organic solvent such as toluene is added thereto to separate the organic layer, which is then washed with water. The organic solvent is distilled off to give methyl 2-(4-methoxycarbonylmethylphenoxymethyl)benzoate represented by Formula (7).

Methyl 4-hydroxyphenylacetate can be prepared by allowing 4-hydroxyphenylacetic acid to react directly with methanol in the presence of an acid catalyst such as sulfuric acid.

### [Step 4] Step of hydrolyzing methyl 2-(4-methoxycarbonylmethylphenoxymethyl)benzoate to produce 2-(4-carboxymethylphenoxymethyl)benzoic acid represented by Formula (8):

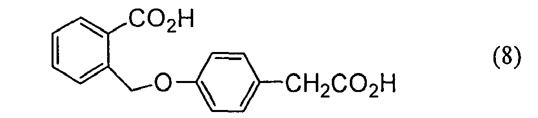

Hydrolysis in this step may be performed in accordance with a known ester hydrolysis method that performs hydrolysis with an acid or an alkali.

### [Step 5] Step of treating 2-(4-carboxymethylphenoxymethyl)benzoic acid with trifluoroacetic anhydride or a halogenating agent and cyclizing with a Lewis acid catalyst to produce a carboxylic acid represented by Formula (1):

This step is usually performed in a solvent, and an inert solvent such as dichlorobenzene and chlorobenzene is used. The solvent is usually used in an amount of about 0.5 to 2 L per 1 mol of the 2-(4-carboxymethylphenoxymethyl) benzoic acid. The temperature treating the 2-(4-carboxymethylphenoxymethyl)benzoic acid with the trifluoroacetic anhydide or the halogenating agent is preferably 10 to 100°C.

The amount of the trifluoroacetic anhydride is preferably about 2 to 2.5 mol per 1 mol of the 2-(4-carboxymethylphenoxymethyl)benzoic acid.

In the treatment of the 2-(4-carboxymethylphenoxymethyl)benzoic acid with the halogenating agent, the halogenating agent reacts with the 2-(4-carboxymethylphenoxymethyl)benzoic acid in an amount of 2 to 2.5 mol per 1 mol of the 2-(4-carboxymethylphenoxymethyl)benzoic acid to produce an acid halide (carboxylic dihalide), and then cyclized with a Lewis acid catalyst such as boron trifluoride, aluminum chloride or the like. Thionyl chloride or the like is preferably used for the halogenating agent.

The amount of the Lewis acid is preferably about 0.05 to 0.2 mol per 1 mol of the 2-(4-carboxymethylphenoxymethyl) benzoic acid. The temperature for the cyclization reaction is preferably -20°C to +30°C.

The tertiary alkyl ester represented by Formula (2) of the invention can be converted to olopatadine after a reaction with 3-dimethylaminopropylmagnesium chloride, and the tertiary alkyl ester is useful as an intermediate of olopatadine useful as a medicament. That is, after the tertiary alkyl ester represented by Formula (2) reacts with the 3-dimethylaminopropylmagnesium chloride, dehydration reaction and de-esterification are performed, whereby olopatadine can be produced. Hereinafter, explanation is given with reference to an example where t-butyl ester is used as a tertiary alkyl ester.

First, t-butyl 11-hydroxy-11-(3'-dimethylaminopropyl)-6,11-dihydrodibenz[b,e]oxepin-2-acetate represented by Formula (9): 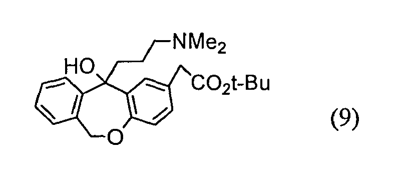 is produced by a reaction of t-butyl ester represented by Formula (3) with 3-dimethylaminopropylmagnesium chloride.

The 3-dimethylaminopropylmagnesium chloride used herein can be produced from, for example, 3-dimethylaminopropyl chloride hydrochloride and magnesium in accordance with a conventional method producing a Grignard reagent. The amount of the 3-dimethylaminopropyl chloride is preferably about 1 to 2 mol per 1 mol of the t-butyl ester represented by Formula (3). The 3-dimethylaminopropyl chloride is preferably used as a solution dissolved in a suitable solvent, such as a mixed solvent of toluene and tetrahydrofuran, to a concentration of about 10 to 40%, and is preferably slowly added dropwise to a solution of the t-butyl ester represented by Formula (3) dissolved in an inert solvent such as tetrahydrofuran. The temperature of the solution is preferably about 10 to 30°C at the dropwise addition.

After the reaction is completed, t-butyl 11-hydroxy-11-(3'-dimethylaminopropyl)-6,11-dihydrodibenz[b,e]oxepin-2-acetate can be obtained from the reaction mixture by conventional procedures such as extraction, phase separation, washing, drying and concentration.

Next, the above-obtained t-butyl 11-hydroxy-11-(3'-dimethylaminopropyl)-6,11-dihydrodibenz[b,e]oxepin-2-acetate is dehydrated and de-esterified. The dehydration reaction and de-esterification may be performed as follows, for example.

Hydrochloric acid having a concentration of about 1 to 35% is added to the t-butyl 11-hydroxy-11-(3'-dimethylaminopropyl)-6,11-dihydrodibenz[b,e]oxepin-2-acetate in an amount of about 1 to 5 mol per 1 mol of the t-butyl 11-hydroxy-11-(3'-dimethyl.aminopropyl)-6,11-dihydrodibenz[b,e]oxepin-2-acetate, and stirred at temperature of about 20 to 100°C for about 0.5 to 10 hours.

### Examples

Hereinafter, the present invention will be described in further detail with reference to the Examples; however, the invention is not limited to these Examples.

### Example 1

### Production of 11-oxo-6,11-dihydrodibenz[b,e]oxepin-2-acetic acid

### • Step 1

To a 1-L four-necked flask, 643 ml of xylene, 134.1 g (1.0 mol) of phthalide, 18.2 g (0.08 mol) of benzyltriethylammonium chloride, and 9.9 g (0.07 mol) of BF₃-etherate complex were added, and heated to 100°C. Subsequently, 142.8 g (1.2 mol) of thionyl chloride was added dropwise thereto over 1 hour, and stirred at 125 to 132°C for 2 hours. Xylene and excessive thionyl chloride (about 350 ml) were distilled off at normal pressure until the internal temperature became 135°C, and xylene (about 350 ml) was further distilled off under reduced pressure.

### • Step 2

The concentrated residue obtained in the above Step 1 was cooled on a water bath, and, while maintaining the internal temperature at not more than 60°C, 80.1 g (2.5 mol) of methanol was added dropwise thereto over 1 hour. The mixture was further stirred at 50°C for 1 hour, and then cooled to 25°C.

Next, 300 ml of toluene was added to the reaction mixture, and the mixture was added dropwise over 30 minutes to a solution of 70.0 g (0.51 mol) of potassium carbonate dissolved in 300 ml of water. The toluene layer was separated, washed with 130 g of 30% brine, and then concentrated under reduced pressure to obtain 189.2 g of methyl 2-chloromethylbenzoate. The apparent yield was 102.5%.

### (Physical Property Data)

MS (m/Z) 184 (M⁺)

### • Step 3

Into a 500-ml four-necked flask, 30.4 g (0.20 mol) of 4-hydroxyphenylacetic acid and 300 ml of methanol were charged, and 0.1 g of sulfuric acid was added thereto. Then, the mixture was stirred while heating and refluxing for 1 hour. Subsequently, methanol was mostly distilled off under reduced pressure. Subsequently, 100 ml of methanol was added thereto, and methanol was distilled off again to prepare methyl 4-hydroxyacetate.

Into a 1-L four-necked flask, 38.8 g (0.21 mol) of the methyl 2-chloromethylbenzoate obtained in the above Step 2, 30.4 g (0.22 mol) of potassium carbonate, and 100 ml of dimethylacetamide were charged and heated to 90°C.

The whole quantity of the methyl 4-hydroxyacetate obtained above was added dropwise to this solution over 1 hour, and then stirred at 90°C for 7 hours. The reaction mixture was poured into 400 ml of water, then 200 ml of toluene was added thereto, and the toluene layer was separated. The obtained toluene layer was washed with 200 ml of water, and then toluene was distilled off to obtain 64.3 g (0.2045 mol) of methyl 2-(4-methoxycarbonylmethylphenoxymethyl)benzoate. The apparent yield was 102.2%.

### (Physical Property Data)

¹H NMR (400 MHz, CDCl₃)
δ 3.57 (s, 2H), 3.68 (s, 3H), 3.90 (s, 3H), 5.49 (s, 2H), 6.95 (d, J = 8.8 Hz, 2H), 7.19 (d, J = 8.4 Hz, 2H), 7.37 (t, J = 7.6 Hz, 1H), 7.74 (d, J = 7.6 Hz, 1H), 8.02 (d, J = 8.0 Hz, 1H)

### • Step 4

The whole quantity of the methyl 2- (4-methoxycarbonylmethylphenoxymethyl)benzoate obtained in the above Step 3 was charged into a four-necked flask. A solution of 17.0 g of sodium hydroxide dissolved in 100 ml of water and 150 ml of methanol were added thereto, and stirred at 65°C for 2 hours. Then, the mixture was cooled to room temperature (around 25°C), and diluted with 200 ml of water. Subsequently, 2.0 g of activated carbon was added thereto, stirred at room temperature for 1 hour, and filtered through a Buchner funnel to separate activated carbon. The activated carbon was washed on a Buchner funnel with 100 ml of water. The filtrate and the cleaning solution were mixed and heated to 60°C, and a solution of 26.5 g of acetic acid dissolved in 50 ml of water was added dropwise thereto over 2 hours to precipitate crystals at the same temperature. Subsequently, the mixture was cooled to 10°C, filtered through a Buchner funnel to collect the crystals, and washed with 200 ml of water. Washed crystals were dried under reduced pressure to obtain 46.5 g (0.1624 mol) of 2-(4-carboxymethylphenoxymethyl)benzoic acid. The yield (yield from the 4-hydroxyphenylacetic acid of Step 3) was 81.2%.

### (Physical Property Data)

¹H NMR (400 MHz, DMSOd₆)
δ 3.47 (s, 2H), 5.45 (s, 2H), 6.90 (d, J = 8.0 Hz, 2H) 7.16 (d, J = 8.4 Hz, 2H), 7.47 (t, J = 7.6 Hz, 1H), 7.36 (t, J = 7.4 Hz, 1H), 7.57 (d, J = 7.2 Hz, 1H), 7.86 (d, J = 7.6 Hz, 1H)

### • Step 5

The whole quantity of the 2-(4-carboxymethylphenoxymethyl)benzoic acid obtained in the above Step 4 and 200 ml of chlorobenzene were charged into a 500-ml four-necked flask, 75.0 g (0.3753 mol) of trifluoroacetic anhydride was added thereto, and the mixture was stirred at about 20°C for 4 hours. Subsequently, 2.3 g (0.0162 mol) of BF₃-etherate complex was added dropwise thereto at -10 to 0°C over 10 minutes, then the mixture was stirred for 30 minutes. An aqueous phase was then separated, and the organic layer was washed with 200 ml of water. The washed organic layer was added to a solution of 7.2g of sodium hydroxide dissolved in 300 ml of water, and stirred for 30 minutes. An aqueous phase was then separated. To the separated aqueous layer, 2.0 g of activated carbon was added, stirred for 30 minutes, and filtered though a Buchner funnel to separate activated carbon. The activated carbon was washed on a Buchner funnel with 10 ml of water. A mixture of the filtrate and the cleaning solution was maintained at about 40°C, and a mixed solution of 11.3 g (0.1876 mol) of acetic acid and 50 ml of water was added dropwise thereto over 30 minutes. After the dropwise addition was completed, the mixture was cooled to 0 to 10°C, filtered through a Buchner funnel to collect crystals, and washed with 200 ml of water. The washed crystals were dried under reduced pressure to obtain 42.0 g of the title compound, (11-oxo-6,11-dihydrodibenz[b,e]oxepin-2-acetic acid). The yield (yield from 2-(4-carboxymethylphenoxymethyl)benzoic acid) was 96.4%, and the purity as measured by HPLC was 99.9%.

### (HPLC conditions)

Column: Inertsil ODS-5 µm (4.6 mm ID x 15 cm)
Mobile Phase: 0.02% trifluoroacetic acid aqueous solution/acetonitrile = 5/5 → 3/7 (30 minutes)
Detection Wavelength: UV 254 nm

### (Physical Property Data)

¹H NMR (400 MHz, DMSOd₆)
δ 3.63 (s, 2H), 5.30 (s, 2H), 7.07 (d, J = 8.0 Hz, 2H), 7.48 (t, J = 3.6 Hz, 1H), 7.55 (d-d, J = 7.9 Hz, 2H), 7.67 (t, J = 7.6 Hz, 1H), 7.78 (d, J = 7.6 Hz, 1H), 7.97 (d, J = 2.0 Hz, 1H)

### Example 2

### Production of t-butyl 11-oxo-6,11-dihydrodibenz[b,e] oxepin-2-acetate

Into a 1-L four-necked flask, 60.4 g (0.225 mol) of the 11-oxo-6,11-dihydrodibenz[b,e]oxepin-2-acetic acid obtained according to the method of Example 1 and 300 ml of toluene were charged, 49.6g (0.236 mol) of trifluoroacetic anhydride was added thereto, and the mixture was stirred at about 20°C for 1 hour. Then, 100 ml of t-butanol was added thereto, stirred at about 20°C for 2 hours, and further stirred at 80°C for 2 hours. The mixture was cooled to about 20°C, 600 ml of water was added thereto and stirred for 20 minutes, and then an organic layer was separated. The organic layer was washed with 400 ml of water, and then washed with a solution of 6.2g (0.045 mol) of potassium carbonate dissolved in 100 ml of water. To the washed organic layer, 3.0 g of activated carbon was added, stirred, and then filtered though a Buchner funnel to separate activated carbon. The activated carbon was washed on a Buchner funnel with 50 ml of toluene. The filtrate and the cleaning solution were mixed, and concentrated under reduced pressure to obtain 58.3 g of the title compound. The apparent yield was 79.9%, and the purity as measured by HPLC was 99.1%.

### (HPLC conditions)

Column: Inertsil ODS-5 µm (4.6 mm ID x 15 cm)
Mobile Phase: 0.02% trifluoroacetic acid aqueous solution/acetonitrile = 5/5 → 3/7 (30 minutes)
Detection Wavelength: UV 254 nm

### (Physical Property Data)

¹H NMR (400 MHz, CDCl₃)
δ 1.45 (s, 9H), 3.55 (s, 2H), 5.17 (s, 2H), 7.02 (d, J = 8.4, 1H), 7.40-7.48 (m, 3H), 7.54 (t, J = 6.4 Hz, 1H), 7.89 (d, J = 6.4 Hz, 1H), 8.11 (d, J = 2.4 Hz, 1H)

### Example 3

### Production of t-butyl 11-oxo-6,11-dihydrodibenz[b,e] oxepin-2-acetate

Into a 3-L four-necked flask, 200 g (0.746 mol) of 11-oxo-6,11-dihydrodibenz[b,e]oxepin-2-acetic acid and 1500 ml of chlorobenzene were charged, 45.8 g (0.298 mol) of phosphorus oxychloride and 2.7 g (0.149 mol) of water were added thereto, and the mixture was heated to 40°C. Then, 209.2 g (3.728 mol) of isobutene was blown thereinto over about 8 hours. The reaction mixture was poured, with cooling, into an aqueous solution of 206.2 g (1.492 mol) of potassium carbonate dissolved in 800 ml of water, and stirred at about 25°C for 1 hour. An organic layer was then separated. Further, the organic layer was washed with an aqueous solution of 51.5 g (0.323 mol) of potassium carbonate dissolved in 200 ml of water, and then concentrated under reduced pressure to obtain 243 g of crude oil of the title compound.

The obtained crude oil was dissolved in 500 ml of methanol, and 10 g of activated carbon was added thereto, stirred at 50°C for 1 hour, and filtered through a Buchner funnel. The activated carbon was then washed with 50 ml of methanol heated to 50°C. The filtrate and the cleaning solution were mixed, and slowly cooled, whereby crystals were precipitated at 30°C. It was further cooled to 10°C, and then filtered through a Buchner funnel. The crystals were washed with 15 ml of methanol cooled to 10°C. The obtained crystals were dried under reduced pressure to obtain 206.2 g (0.636 mol) of the title compound. The yield was 85.3%, and the purity as measured by HPLC was 99.4%. Melting point 68.8°C.

### Example 4

### Production of t-butyl 11-oxo-6,11-dihydrodibenz[b,e] oxepin-2-acetate

Into a 500-ml four-necked flask, 100 ml of toluene was charged, cooled to 0°C, and 11.2g (0.20 mol) of isobutene was blown thereinto. To this solution, 13.4 g (0.05 mol) of the 11-oxo-6,11-dihydrodibenz[b,e]oxepin-2-acetic acid obtained according to the method of Example 1 was added, and stirred at 0°C. Further, 1.53 g (0.01 mol) of phosphorus oxytrichloride was added to this solution, then the temperature was slowly raised to room temperature (about 25°C), and subsequently 0.09 g of water was added thereto. Subsequently, the mixture was heated to 40°C in an autoclave, and stirred for 5 hours to react. The reaction mixture was poured to a solution of 13.8 g (0.10 mol) of potassium carbonate dissolved in 100 ml of water, and stirred at room temperature (about 25°C) for 30 minutes. Then, the toluene layer was separated, dried over 2.0 g of magnesium sulfate, and filtered through a Buchner funnel. The filtrate was concentrated under reduced pressure to obtain 10.8 g (0.0033 mol) of the title compound. The apparent yield was 66.7%, and the purity as measured by HPLC was 99.5%.

### (HPLC conditions)

Column: Inertsil ODS-5 µm (4.6 mm ID x 15 cm)
Mobile Phase: 0.02% trifluoroacetic acid aqueous solution/acetonitrile = 5/5 → 3/7 (30 minutes)
Detection Wavelength: UV 254 nm

### (Physical Property Data)

¹H NMR (400 MHz, CDCl₃)
δ 1.45 (s, 9H), 3.55 (s, 2H), 5.17 (s, 2H), 7.02 (d, J = 8.4, 1H), 7.40-7.48 (m, 3H), 7.54 (t, J = 6.4 Hz, 1H), 7.89 (d, J = 6.4 Hz, 1H), 8.11 (d, J = 2.4 Hz, 1H)

### Example 5

### Production of 11-(3'-dimethylaminopropylidene)-6,11-dihydrodibenz[b,e]oxepin-2-acetic acid

Into a four-necked flask, 6.49 g (0.02 mol) of the t-butyl (11-oxo-6,11-dihydrodibenz[b,e]oxepin-2-yl)acetate obtained according to the same method as in Example 1 and 50 ml of tetrahydrofuran were charged. A mixed solvent solution of toluene (15.4 ml)/tetrahydrofuran (11.7 ml) containing 0.03 mol equivalent amount of 3-dimethylaminopropylmagnesium chloride was added dropwise thereto at 15 to 20°C over 2.5 hours. After the dropwise addition was completed, the mixture was stirred for 30 minutes, and the disappearance of ingredients was confirmed by HPLC. The reaction mixture was added to a mixed solution of 30 ml of water and 5.4 g of acetic acid, and the pH was adjusted to 9.6 with 28% aqueous ammonia. An aqueous phase was separated therefrom. The organic layer was washed with 50 ml of 15% brine, and concentrated to obtain t-butyl 11-hydroxy-11-(3'-dimethylaminopropyl)-6,11-dihydrodibenz[b,e]oxepin-2-acetate at a yield of 97.7%.

### (Physical Property Data)

¹H NMR (400 MHz, CDCl₃)
δ 1.41 (s, 9H), 1.43-1.45 (m, 2H) 1.96 (q, J = 8.4 Hz, 1H), 2.19-2.26 (m, 2H), 2.26 (s, 6H), 3.20 (q, J = 8.0 Hz, 1H), 3.48 (s, 1H), 5.02 (d, J = 15.6 Hz, 1H), 5.45 (d, J = 15.6 Hz, 1H), 6.87 (d, J = 6.8 Hz, 1H), 7.03 (d, J = 7.6 Hz, 1H), 7.13-7.16 (m, 2H), 7.23 (t, J = 8.0 Hz, 2H), 7.67 (d, J = 2.0 Hz, 1H), 8.08 (d, J = 9.6 Hz*,* 1H)

Into a four-necked flask, 8.04 g (0.02 mol) of the t-butyl 11-hydroxy-11-(3'-dimethylaminopropyl)-6,11-dihydrodibenz[b,e]oxepin-2-acetate obtained according to the above method, 4.2 g (0.04 mol) of 35% hydrochloric acid, and 16.0 ml of toluene were charged and stirred at 100°C for 6 hours. The disappearance of ingredients was confirmed by HPLC, and the title compound was thus obtained.

### Industrial Applicability

The tertiary alkyl ester of the invention is easily manufacturable, and is useful as an intermediate that can be converted to olopatadine after a reaction with 3-dimethylaminopropylmagnesium chloride.

## Claims

1. A tertiary alkyl ester represented by Formula (2): 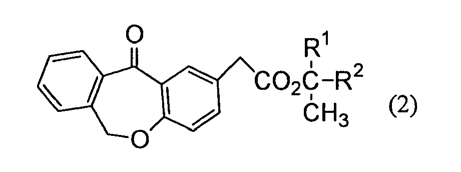 wherein R¹ and R² each independently represent a C₁₋₄ alkyl group.

2. A tertiary alkyl ester according to claim 1, wherein R¹ and R² each represent a methyl group.

3. A method for producing a tertiary alkyl ester represented by Formula (2): 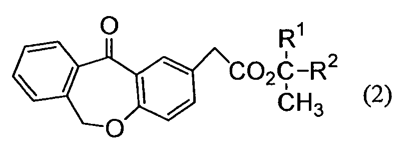 wherein R¹ and R² each independently represent a C₁₋₄ alkyl group, the method comprising esterifying a carboxylic acid represented by Formula (1): 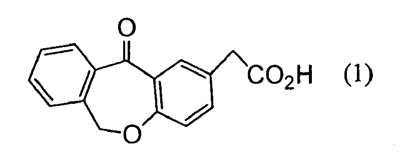 with trifluoroacetic anhydride and a tertiary alcohol.

4. A method for producing a tertiary alkyl ester represented by Formula (2): 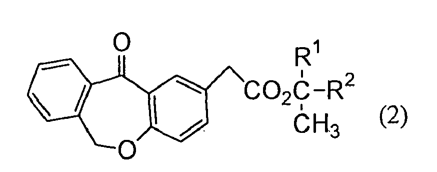 wherein R₁ and R₂ each independently represent a C₁₋₄ alkyl group, the method comprising esterifying a carboxylic acid represented by Formula (1): 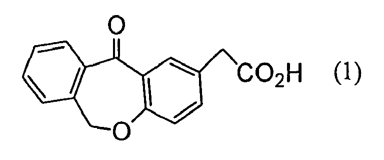 with a vinylidene compound represented by Formula (4): 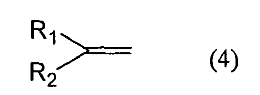 wherein R¹ and R² each independently represent a C₁₋₄ alkyl group, and a phosphorus halide.

5. A method for producing a tertiary alkyl ester represented by Formula (2): 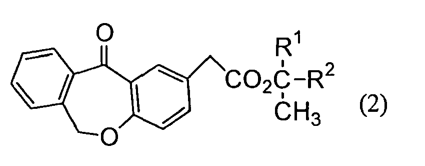 wherein R¹ and R² each independently represent a C₁₋₄ alkyl group, the method comprising:
[Step 1] a step of a reaction of phthalide with thionyl chloride to produce 2-chloromethylbenzoyl chloride;
[Step 2] a step of a reaction of the 2-chloromethylbenzoyl chloride with methanol to produce methyl 2-chloromethylbenzoate represented by Formula (5): 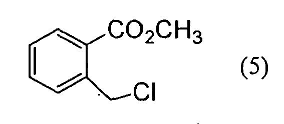
[Step 3] a step of a reaction of the methyl 2-chloromethylbenzoate with methyl 4-hydroxyphenylacetate represented by Formula (6): 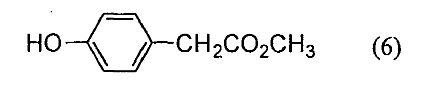 to produce methyl 2-(4-methoxycarbonylmethylphenoxymethyl)benzoate represented by Formula (7): 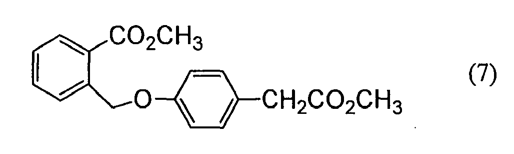
[Step 4] a step of hydrolyzing the methyl 2-(4-methoxycarbonylmethylphenoxymethyl)benzoate to produce 2-(4-carboxymethylphenoxymethyl)benzoic acid represented by Formula (8): 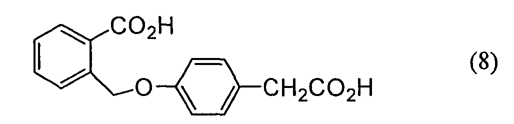
[Step 5] a step of treating the 2-(4-carboxymethylphenoxymethyl)benzoic acid with trifluoroacetic anhydride or a halogenating agent and cyclizing with a Lewis acid catalyst to produce a carboxylic acid represented by Formula (1), and
[Step 6] esterifying the carboxylic acid represented by Formula (1) by a method according to claim 3.

6. A method for producing a tertiary alkyl ester represented by Formula (2): 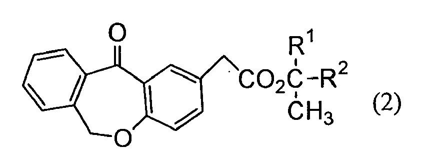 wherein R¹ and R² each independently represent a C₁₋₄ alkyl group, the method comprising:
[Step 1] a step of a reaction of phthalide with thionyl chloride to produce 2-chloromethylbenzoyl chloride;
[Step 2] a step of a reaction of the 2-chloromethylbenzoyl chloride with methanol to produce methyl 2-chloromethylbenzoate represented by Formula (5): 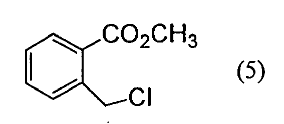
[Step 3] a step of a reaction of the methyl 2-chloromethylbenzoate with methyl 4-hydroxyphenylacetate represented by Formula (6): 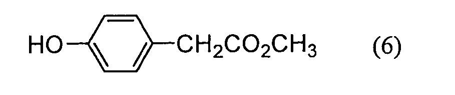 to produce methyl 2-(4-methoxycarbonylmethylphenoxymethyl) benzoate represented by Formula (7): 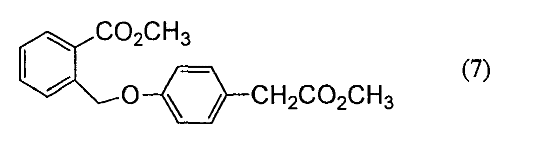
[Step 4] a step of hydrolyzing the methyl 2-(4-methoxycarbonylmethylphenoxymethyl)benzoate to produce 2-(4-carboxymethylphenoxymethyl)benzoic acid represented by Formula (8): 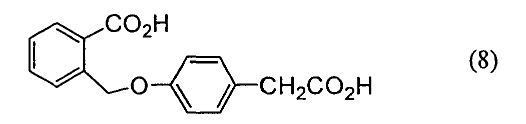
[Step 5] a step of treating the 2-(4-carboxymethylphenoxymethyl)benzoic acid with trifluoroacetic anhydride or a halogenating agent and cyclizing with a Lewis acid catalyst to produce a carboxylic acid represented by Formula (1); and
[Step 6] esterifying the carboxylic acid represented by Formula (1) by a method according to claim 4.

7. A method for producing olopatadine, the method comprising steps of a reaction of t-butyl ester represented by Formula (3): 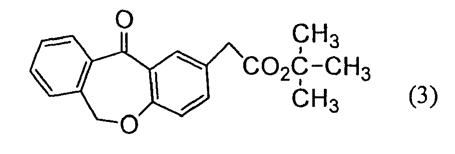 with 3-dimethylaminopropylmagnesium chloride to produce t-butyl 11-hydroxy-11-(3'-dimethylaminopropyl)-6,11-dihydrodibenz[b,e]oxepin-2-acetate represented by Formula (9) : 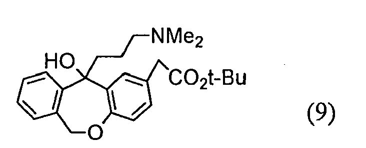 and then subjecting the same to dehydration reaction and de-esterification reaction.
